# EUROPÄISCHE PATENTSCHRIFT

(11) **EP 0 724 416 B1**
(45) Veröffentlichungstag und Bekanntmachung des Hinweises auf die Patenterteilung: **09.02.2000**
(21) Anmeldenummer: 94928361.8
(22) Anmeldetag: 15.09.1994
(51) Int. Cl.: A61F 13/15

(54) **WEGWERFWINDEL**
DISPOSABLE NAPPY
COUCHE JETABLE

(30) Priorität: 18.10.1993 DE 4335443
(43) Veröffentlichungstag der Anmeldung: 07.08.1996
(73) Patentinhaber: Paul Hartmann Aktiengesellschaft, 89522 Heidenheim (DE)
(72) Erfinder: MALOWANIEC, Krzysztof, D., D-89522 Heidenheim (DE)
(74) Vertreter: Dreiss, Fuhlendorf, Steimle & Becker, Patentanwälte
(86) Internationale Anmeldenummer: EP9403094
(87) Internationale Veröffentlichungsnummer: WO9510993

(56) Entgegenhaltungen:
- EP-A- 0 263 720
- EP-A- 0 539 703
- EP-A- 0 563 971
- WO-A-94/03136
- US-A- 4 662 877

## Beschreibung

Die Erfindung betrifft eine Wegwerfwindel, wie sie im Oberbegriff des Patentanspruchs 1 beschrieben ist. Diese Wegwerfwindel weist auf der dem Körper zugewandten Seite eine hydrophobe, mit einer Öffnung versehene Vliesstoffauflage auf.

Aus der EP-A-0 263 720 ist eine Wegwerfwindel bekannt, die an ihren Längs- und Taillenrändern Dichtklappen aufweist, die in den Randbereichen des Saugkörpers der Windel angeordnet sind. Der Saugkörper besteht aus einer Lage aus Zellstoff-Fluff und weist eine Umhüllung aus Papier auf, damit der Saugkörper bei einem Flüssigkeitsanfall nicht auseinanderfällt oder zusammenklumpt.

In der US-PS 4,662,877 ist eine Wegwerfwindel beschrieben, die eine Wäscheschutzfolie, eine flüssigkeitsdurchlässige Vliesstoffabdeckung, einen zwischen Vliesstoffabdeckung und Wäscheschutzfolie befindlichen rechteckigen Saugkörper und eine rechteckige hydrophobe Vliesstoffauflage aufweist. Die hydrophobe Vliesstoffauflage ist auf die flüssigkeitsdurchlässige Vliesstoffabdeckung, die kantenbündig mit der Wäscheschutzfolie abschließt, aufgebracht.

Ausgehend von diesem Stand der Technik ist es Aufgabe der Erfindung, die bekannte Wegwerfwindel derart zu verbessern, daß insbesondere Körperausscheidungen schneller aufgenommen und in der Windel besser gehalten werden.

Die Aufgabe wird erfindungsgemäß gelöst durch die kennzeichnenden Merkmale des Patentanspruchs 1. Diese Wegwerfwindel hat den Vorteil, daß die Verteilerschicht die anfallenden Körperflüssigkeiten sehr schnell aufnimmt und über die Schicht verteilt. Die Saugschicht nimmt dann die Flüssigkeit aus der Verteilerschicht, die nur ein geringes Flüssigkeitsrückhaltevermögen hat und als voluminöses Vlies mit einem Flächengewicht von 25 bis 120 g/m² ausgebildet ist, auf und speichert diese. Befindet sich bei Druckbelastung auf den Saugkörper noch Flüssigkeit in der Verteilerschicht, so wird diese aus der Verteilerschicht herausgedrängt. Die Verdrängung der Flüssigkeit wird insbesondere auch am Rand der Verteilerschicht erfolgen, da die Saugschicht die Flüssigkeit nicht schnell genug aufsaugen und speichern kann. Da nun erfindungsgemäß die Abmessungen der Öffnung der Vliesstoffauflage in Windellängs- und Windelquerrichtung kleiner sind als die entsprechenden Abmessungen der Verteilerschicht, und da die hydrophobe Vliesstoffauflage mit der Vliesstoffabdeckung innerhalb des Randes der Verteilerschicht verbunden ist, wird die herausgedrängte Flüssigkeit durch die ersten und zweiten Dichtklappen aufgefangen und kann von der Saugschicht oder der Verteilerschicht selbst sofort wieder absorbiert werden. Sie kann somit nicht aus der Windel austreten.

Die Verteilerschicht wirkt damit wie ein Schwamm, wobei die Flüssigkeit so lange durch die Dichtklappen gehalten ist, bis die Saugschicht die Flüssigkeit vollständig aufgenommen hat.

Vorteilhafte Ausgestaltungen sind Gegenstand der Unteransprüche.

So ist es vorgesehen, daß die hydrophobe Vliesstoffauflage kantenbündig mit der Wäscheschutzfolie abschließt und im Randbereich mit dieser verbunden ist, also die flüssigkeitsdurchlässige Vliesstoffabdeckung des Saugkörpers in Breite und Länge kleiner ist als die Wäscheschutzfolie und die hydrophobe Vliesstoffauflage. Ein Kriechen der Flüssigkeit nach außen zum Windelrand entlang der flüssigkeitsdurchlässigen Vliesstoffabdeckung nach Art einer Dochtwirkung ist damit verhindert.

Die Saugschicht des Saugkörpers besteht aus Zellstoff-Fluff, der mit superabsorbierendem Material vermischt sein kann. Der Gehalt an superabsorbierendem Material ist relativ hoch und beträgt vorzugsweise 15-70 %, um der Saugschicht ein hohes Flüssigkeitsrückhaltevermögen zu verleihen.

Als Verteilerschicht kann ein voluminöses Vies dienen, wie es aus der EP 0 539 703 A1 bekannt ist, oder eine Schicht aus versteiften, gekräuselten, vernetzten Zellulosefasern, wie sie aus der EP 0 252 650 B1 bekannt sind.

Im folgenden wird die Erfindung anhand der Figuren 1 bis 3, die eine bevorzugte Ausgestaltung der Erfindung zeigen, im näheren beschrieben. Es zeigen:
- Figur 1: eine Draufsicht auf eine flachgelegte Wegwerfwindel, bei der ein Teil weggebrochen ist;
- Figur 2: einen Schnitt entlang der im Schrittbereich verlaufenden Linie II-II der Figur 1, wobei die Wegwerfwindel in Gebrauchslage dargestellt ist; und
- Figur 3: einen Schnitt entlang der mit der Längsachse identischen Linie III-III der Figur 1.

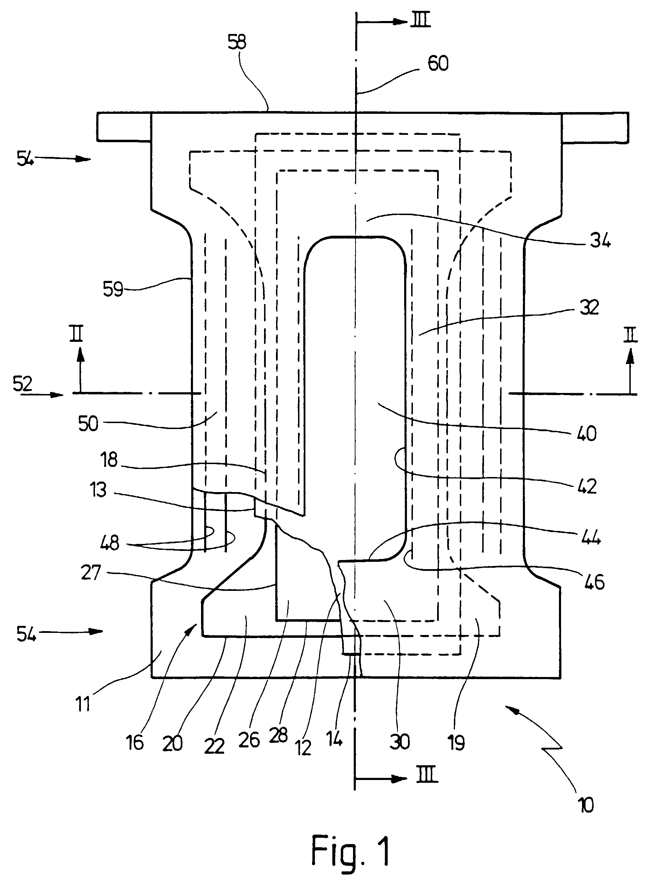

Figur 1 zeigt eine bevorzugte Ausgestaltung einer erfindungsgemäßen Wegwerfwindel 10 im flachgelegten Zustand mit gestreckter Lage der Elastifizierungsmittel.

Die Windel 10 ist bezüglich ihrer Längsachsen 60 symmetrisch und teilt sich in Längsrichtung auf in zwei Taillenbereiche 54 und einen Schrittbereich 52. Gleiche, symmetrisch zueinander liegende Windelelemente sind jeweils nur einmal mit Bezugsziffern gekennzeichnet.

Die Windel 10 weist eine flüssigkeitsundurchlässige, sanduhrförmig zugeschnittene Wäscheschutzfolie 11 und einen darauf liegenden Saugkörper 16 auf. Der Saugkörper 16 ist auf der Wäscheschutzfolie 11 durch eine flüssigkeitsdurchlässige Vliesstoffabdeckung 12, die die Längsseiten 18 und Stirnseiten 20 des Saugkörpers 16 überragt, festgelegt.

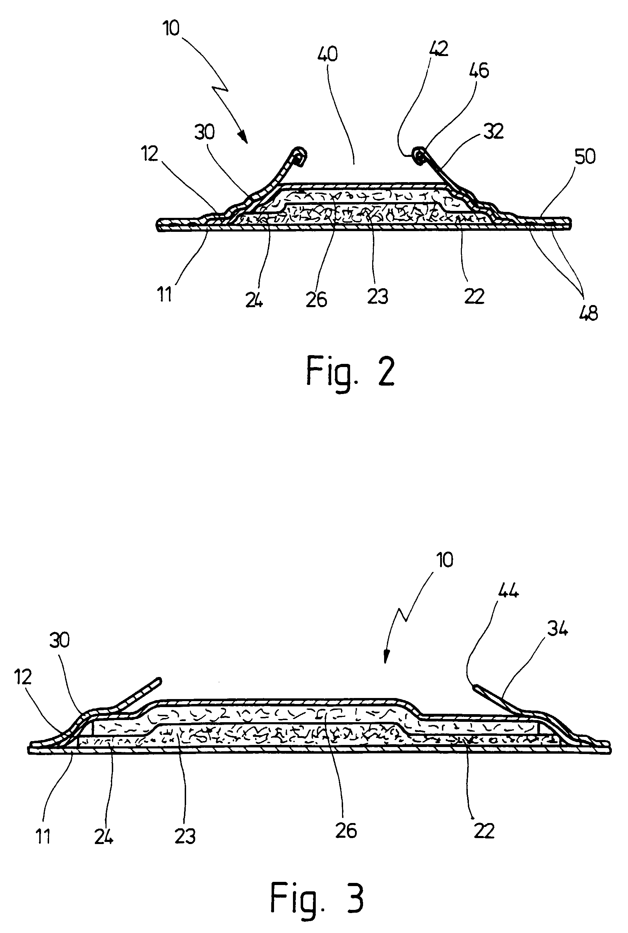

Wie in Figur 2 und 3 dargestellt, weist der Saugkörper 16 eine auf der Wäscheschutzfolie 11 aufliegende Saugschicht 22 und eine auf der Saugschicht liegende Verteilerschicht 26 auf. Die Saugschicht 22 besteht aus einer Mischung aus Zellstoffasern und superabsorbierendem Material, wobei der Anteil an superabsorbierendem Material etwa 15-70 Gew.-% beträgt. In dem in den Figuren 2 und 3 dargestellten Ausführungsbeispiel ist vorgesehen, daß die Saugschicht 22 einen zentralen Bereich 23 mit einem größeren Flächengewicht und größerer Dichte als in den Randbereichen 24 der Saugschicht 22 aufweist. Der zentrale Bereich 23 der Saugschicht 22 ist im Bereich des größten Flüssigkeitsanfalles positioniert. Die Verteilerschicht 26 ist im wesentlichen rechteckförmig und überdeckt den zentralen Bereich 23 der Saugschicht 22.

Die Verteilerschicht 26 kann ein voluminöses Vlies mit einem Flächengewicht von 25-120 g/m² sein, das aus einer Mischung aus Polypropylen- oder Polyesterfasern mit Polyäthylen/Polyester-Bikomponentenfasern besteht. Ein solches Vlies ist aus der EP 0 539 703 A1 bekannt. Die Verteilerschicht 26 kann auch aus Zellulosefasern bestehen, die versteift, gekräuselt und vernetzt sind. Derartige Fasern sind aus der EP 0 252 650 B1 bekannt.

Die den Saugkörper 16 festlegende Vliesstoffabdeckung 12 weist einen rechteckigen Zuschnitt auf und überragt mit ihren Längsrändern 13 die Längsseiten 18 des Saugkörpers 16 im Windelschrittbereich, während sie mit ihren Stirnenden 14 die Stirnseiten 20 des Saugkörpers 16 teilweise überragend mit ihren Rändern mit der Wäscheschutzfolie 11 verbunden ist, mit Ausnahme im Bereich der vier Ecken 19 des Saugkörpers 16. Diese sind nicht von der Vliesstoffabdeckung 12 überdeckt. Die Längsränder 13 der Vliesstoffabdeckung 12 liegen im Abstand von den Seitenrändern 50 der Wäscheschutzfolie 11. Die Stirnenden 14 der Vliesstoffabdeckung 12 sind entweder kantenbündig mit einem Taillenrand 58 der Wäscheschutzfolie oder liegen vorzugsweise, wie in Figur 1 dargestellt, parallel zu den Seitenrändern innerhalb des Taillenrandes 58.

Die Wäscheschutzfolie 11, der Saugkörper 16 und die Vliesstoffabdeckung 12 werden von einer hydrophoben Vilesstoffauflage 30 überdeckt. Diese Vliesstoffauflage 30 hat die gleichen Außenabmessungen wie die Wäscheschutzfolie 11, so daß sie kantenbündig mit dieser abschließt. Die hydrophobe Vliesstoffauflage 30 ist mit dem Saugkörper 16 und der Wäscheschutzfolie 11 wenigstens an den Flächen verbunden, die von der flüssigkeitsdurchlässigen Vliesstoffabdeckung 12 nicht abgedeckt sind. Die hydrophobe Vliesstoffauflage 30 ist innerhalb der Längsseiten 27 und Stirnseiten 28 der Verteilerschicht 26 mit der flüssigkeitsdurchlässigen Vliesstoffabdeckung 12 verbunden.

Die Vliesstoffauflage 30 weist eine zentrale Öffnung 40 auf, die durch freie Längsränder 42 und Stirnseiten 44 begrenzt ist. Entlang der Längsränder 42 sind Elastifizierungsmittel 46 vorgesehen, deren wirksame Strecke über die Stirnseiten 44 der Öffnung 40 hinausgeht. Die Elastifizierungsmittel 46 befinden sich auf der dem Saugkörper zugewandten Seite der Vliesstoffauflage 30 und werden durch ein Umklappen des Längsrandes 42 der Öffnung umhüllt, wie in Figur 2 dargestellt. Die Elastifizierungsmittel 46 können durch einen zusätzlichen Streifen Vlies abgedeckt oder in jeweils eine sich über die gesamte Länge der hydrophoben Vliesstoffauflage 30 erstreckende Falte eingebracht sein. Dadurch wird ein unangenehmer Kontakt zwischen Elastifizierungsmitteln 46 und der Haut verhindert. Auch die Querränder der Öffnung können mit Elastifizierungsmitteln ausgestattet sein.

In Gebrauchslage der Wegwerfwindel 10 kontrahieren die Elastifizierungsmittel 46 und bewirken damit ein Abstehen der Längsränder 42 der Öffnung 40 sowie abstehende Stirnseiten 44. Auf diese Weise sind erste Dichtklappen 32 entlang der Längsseite 27 der Verteilerschicht 26 (Figur 2) sowie zweite Dichtklappen 34 (Figur 3) an den Stirnseiten gebildet.

Da die hydrophobe Vliesstoffauflage 30 innerhalb des Randes der Verteilerschicht 26 mit der flüssigkeitsdurchlässigen Vliesstoffabdeckung 12 verbunden ist, muß jeder Flüssigkeitsanfall zunächst von der Verteilerschicht aufgenommen werden. Die Dichtklappen 32 und 34 halten die Körperausscheidungen im Bereich der Verteilerschicht 26 des Saugkörpers 16. Die freien Ränder der Dichtklappen 32 und 34, die durch die Ränder 42 und 44 der Öffnung 40 gebildet sind, wirken abdichtend gegen den Körper eines Trägers.

Die Öffnung 40 der Vliesstoffauflage 30 kann nicht nur eine abgerundete rechteckige Form, wie in Figur 1 dargestellt, aufweisen, sondern es sind auch andere Formen möglich. So kann die Öffnung 40 beispielsweise auch eine ovale Form haben, wobei sich die längere Achse in Windellängsrichtung 60 erstreckt. Die Elastifizierungsmittel 46 sollten entlang der Längsseitenränder 42 verlaufen. Es kann statt der dargestellten Form auch eine trapezförmige Öffnung 40 oder eine nicht zentral in der Vliesstoffauflage 30 positionierte Öffnung vorgesehen sein. Dabei ist aber darauf zu achten, daß die Dichtklappen 32 und 34 derart positioniert sind, daß die anfallende Flüssigkeit von der Verteilerschicht 26 des Saugkörpers 16 aufgenommen wird.

Zwischen Vliesstoffauflage 30 und Wäscheschutzfolie 11 befinden sich außerhalb des Längsrandes 13 der Vliesstoffabdeckung 12 parallel zu den Seitenrändern 59 jeweils zwei elastische Bänder 48, die mit der Wäscheschutzfolie 11 und/oder der Vliesstoffauflage 30 verbunden sind. So sind im Windelschrittbereich 52 elastifizierte Seitenlappen 50 gebildet, die eine zusätzliche Abdichtung gegen die Beine eines Trägers bewirken.

## Patentansprüche

1. Wegwerfwindel mit
- einer flüssigkeitsundurchlässigen Wäscheschutzfolie (11),
- einer mit dieser verbundenen, flüssigkeitsdurchlässigen Vliesstoffabdeckung (12),
- einem zwischen Wäscheschutzfolie (11) und Vliesstoffabdeckung (12) befindlichen Saugkörper (16),
- einer auf der dem Körper zugewandten Seite der Windel aufgebrachten, hydrophoben, flüssigkeitsdichten Vliesstoffauflage (30), die mit einer Öffnung (40) im Windelschrittbereich (52) versehen ist,
- wobei die in Windellängsrichtung verlaufenden Randbereiche der Öffnung (40) ein erstes Paar Dichtklappen (32) bilden und die im Windelendbereich liegenden Randbereiche dieser Öffnung (40) ein zweites Paar Dichtklappen (34), die quer zu den ersten Dichtklappen (32) verlaufen, bilden,
**dadurch gekennzeichnet, dass**
der Saugkörper (16) eine Saugschicht (22) und auf der körperzugewandten Seite eine als voluminöses Vlies mit einem Flächengewicht von 25-120 g/m² und geringerem Flüssigkeitsrückhaltevermögen als die Saugschicht (22) ausgebildete Verteilerschicht (26) aufweist, dass die Abmessungen der Öffnung (40) der Vliesstoffauflage (30) in Windellängs- und Windelquerrichtung kleiner sind als die entsprechenden Abmessungen der Verteilerschicht (26) und dass die hydrophobe Vliesstoffauflage (30) mit der Vliesstoffabdeckung (12) innerhalb des Randes (27, 28) der Verteilerschicht (26) verbunden ist, so dass die Dichtklappen (32 und 34) innerhalb des Randes (27, 28) der Verteilerschicht (26) liegen.

2. Wegwerfwindel nach Anspruch 1, dadurch gekennzeichnet, dass die hydrophobe Vliesstoffauflage (30) kantenbündig mit der Wäscheschutzfolie (11) abschließt und im Randbereich mit dieser verbunden ist.

3. Wegwerfwindel nach einem der vorhergehenden Ansprüche, dadurch gekennzeichnet, dass die Saugschicht (22) eine Mischung aus Zellstoff-Fluff und superabsorbierendem Material aufweist.

4. Wegwerfwindel nach Anspruch 3, dadurch gekennzeichnet, dass der Anteil an superabsorbierendem Material 15 bis 70 Gew.% beträgt.

5. Wegwerfwindel nach einem der vorhergehenden Ansprüche, dadurch gekennzeichnet, dass das Vlies aus Polypropylen- oder Polyesterfasern und Polyethylen/Polyester-Bikomponentenfasern besteht.

6. Wegwerfwindel nach einem der vorhergehenden Ansprüche, dadurch gekennzeichnet, dass die Verteilerschicht (26) aus versteiften, gekräuselten und vernetzten Zellulosefasern besteht.

## Claims

1. A disposable nappy having
- a liquid-impermeable clothing-protection film (11),
- a liquid-permeable nonwoven cover (12) connected to said film,
- an absorbent body (16) located between the clothing-protection film (11) and the nonwoven cover (12),
- a hydrophobic, liquid-tight, nonwoven top layer (30), which is positioned on that side of the nappy which faces the body, and is provided with an opening (40) in the crotch region (52) of the nappy,
- those border regions of the opening (40) which extend in the longitudinal direction of the nappy forming a first pair of sealing flaps (32), and those border regions of this opening (40) which are located in the end region of the nappy forming a second pair of sealing flaps (34) which extend transversely with respect to the first sealing flaps (32),
**characterized in that** the absorbent body (16) has an absorbent layer (22) and, on the side facing the body, a dispersing layer (26) which is constructed as a voluminous nonwoven having a mass per unit area of 25-120 g/m² and a lower liquid-retaining capacity than the absorbent layer (22), in that the dimensions of the opening (40) in the nonwoven top layer (30) are smaller in the longitudinal and transverse directions of the nappy than the corresponding dimensions of the dispersing layer (26), and in that the hydrophobic, nonwoven top layer (30) is connected to the nonwoven cover (12) inside the border (27, 28) of the dispersing layer (26), so that the sealing flaps (32 and 34) are located inside the border (27, 28) of the dispersing layer (26).

2. A disposable nappy according to Claim 1, characterized in that the hydrophobic, nonwoven top layer (30) terminates such that its edges are flush with the clothing-protection film (11) and in that it is connected to said film in the border region.

3. A disposable nappy according to one of the preceding claims, characterized in that the absorbent layer (22) contains a mixture of cellulose fluff and super-absorbent material.

4. A disposable nappy according to Claim 3, characterized in that the proportion of super-absorbent material is 15 to 70 % by weight.

5. A disposable nappy according to one of the preceding claims, characterized in that the nonwoven is composed of polypropylene or polyester fibres and polyethylene/polyester bicomponent fibres.

6. A disposable nappy according to one of the preceding claims, characterized in that the dispersing layer (26) is composed of reinforced, crimped and cross-linked cellulose fibres.

## Revendications

1. Couche jetable, comportant
- une pellicule de protection des vêtements perméable aux liquides (11),
- une chape en étoffe de nappe de fibres (12) perméable aux liquides et reliée à cette dernière,
- un corps absorbant (16) se trouvant entre la pellicule de protection des vêtements (11) et la chape en étoffe de nappe de fibres (12),
- une feuille en étoffe de nappe de fibres (30) qui est hydrophobe, étanche aux liquides, appliquée sur le côté de la couche dirigé vers le corps et pourvue d'une ouverture (40) dans la zone de l'entrejambe de la couche (52),
- dans laquelle les zones du bord de l'ouverture (40) s'étendant dans la direction longitudinale de la couche forment une première paire de volets d'étanchéité (32) et les zones de bord de cette ouverture (40) se trouvant dans la zone d'extrémité de la couche forment une deuxième paire de volets d'étanchéité (34) qui s'étendent perpendiculairement aux premiers volets d'étanchéité (32),
**caractérisée en ce que**
le corps absorbant (16) comporte une couche absorbante (22) et, sur le côté dirigé vers le corps, une couche de répartition (26) sous la forme de nappe de fibres volumineuse présentant un poids spécifique de 25-120 g/m² et dont la capacité de rétention des liquides est plus faible que celle de couche absorbante (22), en ce que les dimensions de l'ouverture (40) de la feuille en étoffe de nappe de fibres (30) sont plus petites, dans la direction longitudinale et dans la direction transversale de la couche, que les dimensions correspondantes de la couche de répartition (26) et en ce que la feuille en étoffe de nappe de fibres (30) hydrophobe est reliée, à l'intérieur du bord (27, 28) de la couche de répartition (26), avec la chape en étoffe de nappe de fibres (12) de telle manière que les volets d'étanchéité (32 et 34) se trouvent à l'intérieur du bord (27, 28) de la couche de répartition (26).

2. Couche jetable selon la revendication 1, caractérisée en ce que la feuille en étoffe de nappe de fibres (30) hydrophobe se termine bord à bord avec la pellicule de protection des vêtements (11) et est reliée à cette dernière dans la zone de bord.

3. Couche jetable selon l'une quelconque des revendications précédentes, caractérisée en ce que la couche absorbante comporte un mélange de duvet en cellulose et de matière superabsorbante.

4. Couche jetable selon la revendication 3, caractérisée en ce que la proportion de matière superabsorbante est comprise entre 15 à 70 % en poids.

5. Couche jetable selon l'une quelconque des revendications précédentes, caractérisée en ce que la nappe de fibres est constituée de fibres de polypropylène ou de fibres de polyester et de fibres à deux composants polyéthylène/polyester.

6. Couche jetable selon l'une quelconque des revendications précédentes, caractérisée en ce que la couche de répartition (26) est constituée de fibres de cellulose rigidifiées, bouclées et réticulées.
